# EUROPEAN PATENT SPECIFICATION

(11) **EP 0 543 937 B1**
(45) Date of publication and mention of the grant of the patent: **31.01.1996**
(21) Application number: 91916548.0
(22) Date of filing: 09.08.1991
(51) Int. Cl.: C07C 53/12, C07C 53/08, C07C 51/573

(54) **PROCESS FOR THE PURIFICATION OF ACETIC ANHYDRIDE AND ACETIC ACID**
VERFAHREN UND REINIGUNG VON ESSIGSÄUREANHYDRID UND ESSIGSÄURE
PROCEDE DE PURIFICATION D'ANHYDRIDE ACETIQUE ET D'ACIDE ACETIQUE

(30) Priority: 15.08.1990 US 567854
(43) Date of publication of application: 02.06.1993
(73) Proprietor: EASTMAN CHEMICAL COMPANY, Kingsport, TN 37660 (US)
(72) Inventor: TENNANT, Brent, Alan, Kingsport, TN 37664 (US); CUSHMAN, Michael, Roy, Kingsport, TN 37660 (US); MONCIER, Regina, Michelle, Bristol, VA 24201 (US); ZOELLER, Joseph, Robert, Kingsport, TN 37660 (US)
(74) Representative: Behrens, Dieter, Dr.-Ing.
(86) International application number: US9105668
(87) International publication number: WO9203403

(56) References cited:
- EP-A- 0 013 551
- EP-A- 0 143 179
- EP-A- 0 217 182
- EP-A- 0 217 191
- DE-A- 3 612 504
- US-A- 4 792 420

## Description

This invention pertains to a novel process for the purification of a mixture of acetic anhydride and acetic acid obtained from carbonylation processes utilizing iodine and/or iodide compounds. More particularly, this invention pertains to the purification of such anhydride/acid mixtures by a sequential peroxide-hydrogen treatment process.

The preparation of acetic anhydride by contacting a mixture comprising methyl iodide and methyl acetate and/or dimethyl ether with carbon monoxide in the presence of a rhodium catalyst has been reported extensively in the patent literature. See, for example, U.S. Patents 3,927,078, 4,046,807, 4,374,070 and 4,559,183 and European Patents 8396 and 87,870. These patents disclose that the reaction rate can be increased if the catalyst system includes a promoter such as certain amines, quaternary ammonium compounds, phosphines and inorganic compounds such as lithium compounds. The crude or partially-refined product obtained from such acetic anhydride processes typically comprises a mixture of acetic anhydride and acetic acid as a result of the use of acetic acid as a process solvent and/or the co-production of acetic acid by including methanol and/or water in the feed to the carbonylation reactor.

The acetic anhydride and acetic acid obtained from the carbonylation processes referred to above must be purified and refined to meet the purity requirements of users thereof. The purity specification most difficult to achieve are iodine concentration, levels of "reducing substances" and color. Typical specifications require an iodine concentration of 20 parts per billion (ppb) or less, a permanganate reducing substances test value of at least 30 minutes according to a modification of the Substances Reducing Permanganate Test, American Chemical Society Specifications published in Reagent Chemicals, 6th Ed., American Chemical Society, Washington, D.C., pp. 66 and 68, and a color of less than 10 according to ASTM D 1209-84. The anhydride/acid mixture obtained from carbonylation processes also poses corrosion problems with respect to the equipment employed in the refining of the mixture. Prior to our invention, the refining equipment either had to be replaced periodically or had to be fabricated from corrosion-resistant materials, both of which constitute a significant economic burden on the use of the carbonylation processes.

Two methods for removing iodine from acetic anhydride produced by the above-described carbonylation processes have been described in the literature. The treatment of carbonylation acetic anhydride with a peroxide is disclosed in German Offen. 3,612,504 and 3,660,329 and European Patent Applications 13,551 and 217,191 (equivalent of South African Application 86/7389). European Patent Applications 143,179 and 217,182 and U.S. Patent 4,792,420 described the hydrogenation of carbonylation acetic anhydride in the presence of a noble metal catalyst. Our investigation of these methods in the purification of mixtures of acetic anhydride and acetic acid has established that neither consistently produces both (1) acetic anhydride having an acceptable iodine and reducing substances levels and (2) acetic acid having an acceptable iodine level and color. We have found that the known purification procedures presented additional complications. For example, the use of the hydrogenation procedure produced acetic acid of poor color quality which continued to discolor upon storage whereas the peroxide treatment rendered the anhydride/acid mixture very corrosive to steel.

The process provided by this invention comprises contacting a mixture of acetic anhydride and acetic acid, obtained by contacting a mixture comprised of methyl iodide and methyl acetate and/or dimethyl ether with carbon monoxide, with (1) a peroxide and (2) hydrogen in the presence of a supported palladium catalyst and subsequently separating the mixture to obtain acetic anhydride and acetic acid, each of which (i) contains 20 parts per billion or less iodine, (ii) passes the American Chemical Society Specifications Permanganate Reducing Substances test and (iii) has a color value of 10 or less according to ASTM D 1209-84. Our improved process also provides an anhydride/acid mixture which produces less corrosion of the distillation equipment used in the subsequent separation of the mixture. The sequence of the treatments is important since performing the hydrogenation treatment first and then the peroxide treatment would produce a more corrosive product and would result in a less efficient system. As stated above, the acetic anhydride/acetic acid mixture which is purified in accordance with our invention may be obtained by carrying out the carbonylation process in the presence of acetic acid or by including in the mixture which is contacted with carbon monoxide methanol and/or water which results in the coproduction of acetic anhydride and acetic acid.

In the production of acetic anhydride in the presence of acetic acid as well as in the co-production of acetic anhydride and acetic acid, the carbonylation effluent is passed through a plurality of flash pots and or columns to separate most of the acetic anhydride and acetic acid from the non-volatile catalyst components and a substantial portion of the low boilers, e.g., methyl acetate, dimethyl ether, methyl iodide and acetone, present in the reactor effluent. See, for example, the operating system described in U.S. Patent 4,374,070. The crude acetic anhydride/acetic acid mixture contains at this point at least 70, preferably at least 85, weight percent acetic anhydride and acetic acid. Other components which may be present in the mixture include methyl iodide, methyl acetate, acetone, acetyl iodide, ethylidene diacetate, etc. Typically, the mixture contains 1 to 2 weight percent methyl iodide, 5 to 20 weight percent methyl acetate, up to 5 weight percent acetone, 100 to 300 parts per million (ppm) acetyl iodide and up to 2 weight percent ethylidene diacetate.

This crude mixture is treated further in distillation apparatus, e.g., a low boiler column operated at a base temperature of 120 to 140°C and a pressure of 1 to 2 bar, to remove essentially all of the above-mentioned low boilers. The resulting low boiler depleted anhydride/acid mixture is then submitted to the purification process provided by this invention. The acetic anhydride:acetic acid weight ratio of the mixture used in our process may be 4:1 to 1:1 although a weight ratio of 3:1 to 11:7 anhydride:acid normally is used. The mixture normally contains at least 99 weight percent acetic anhydride and acetic acid, a minor amount of ethylidene diacetate and a trace amount of low boilers. The iodine content of the mixture is 3 to 90 ppm iodine, primarily in the form of acetyl iodide.

While hydrogen peroxide, because of its cost, is the preferred peroxide, other peroxides such as diacetyl peroxide and, especially, peracetic acid, may be used in the peroxide treatment step of our novel process. A particularly suitable source of peracetic acid is described as a part of the epoxidation process described by J. T. Lutz, Jr. in Kirk-Othmer, Encyclopedia of Chemical Technology, 3rd Rd., Vol. 9, pp. 225-258 (1980). In the epoxidation process, peracetic acid is generated by contacting acetic acid with hydrogen peroxide in the presence of an acidic ion exchange resin. The amount of peroxide required may vary considerably depending on the particular peroxide and grade thereof used, the composition and iodine content of the mixture, etc. However, the minimum amount used normally is 1 mole of peroxide per equivalent of iodine or iodide, calculated as I⁻, present in the mixture. The upper limit on the amount of peroxide used is determined by economic and safety considerations. The amount of peroxide used preferably is 0.001 to 0.1 moles, especially 0.005 to 0.02 moles, peroxide per liter of mixture.

The conditions of temperature and pressure employed in the peroxide treatment are, in general, not critical but are dictated by the conditions existing at or near the equipment, e.g., a low boiler distillation column, from which the mixture is obtained and the temperatures employed in the hydrogenation step and in the downstream refining equipment. The peroxide treatment therefore may be carried out at a temperature in the range of 50 to 200°C, the preferred range being 110 to 150°C. The pressure may be varied considerably, e.g. from 0.05 to 10 bar although in practical operations the pressure will be determined by the downstream distillation units. Thus, the peroxide treatment preferably is conducted at a pressure of 0.5 to 2.5 bar.

The hydrogen treatment (hydrogenation) is carried out in the liquid phase in the presence of a supported palladium catalyst, e.g., palladium on carbon, in which palladium constitutes 0.1 to 10 weight percent of the catalyst. The catalyst may be employed as a slurry or, preferably, as a fixed bed wherein the anhydride/acid mixture flows over or through the catalyst bed in a mode commonly referred to as trickle bed operation. The amount of catalyst employed depends primarily upon the mode of operation, contact time, and conditions being used and may be determined from the purity of the anhydride and acid ultimately recovered from the process. Due to their cost, the preferred catalyst is palladium on carbon wherein palladium constitutes 0.2 to 2.0 weight percent of the supported catalyst.

The hydrogen treatment may be performed using either pure, or essentially pure, hydrogen or mixtures of hydrogen and an inert gas. For example, mixtures of one or more inert gases, e.g., nitrogen, argon, helium, etc., and 10 or more volume percent hydrogen may be used.

As in the case of the peroxide treatment, the pressure and temperature at which the hydrogen treatment is performed are not critical to the successful operation of the process but depend primarily on the conditions existing in the distillation column used in conjunction with our purification process. Although the hydrogenation may be conducted at 25 to 225°C and 0.05 to 50 bar, the conditions existing in the surrounding equipment favor the use of temperatures in the range of 110 to 150°C and total pressures in the range of 0.5 to 2.5 bar.

In commercial operation, a peroxide such as hydrogen peroxide is added continuously to the underflow of a distillation column from which most of the low boilers such as methyl acetate, methyl iodide, acetone, etc. are removed overhead. The combining of the underflow mixture and peroxide may employ an agitated vessel or simply a conduit which provides sufficient contact between the mixture of acetic anhydride and acetic acid and the peroxide. The resulting mixture then is fed directly to a columnar vessel having fixed therein a bed or beds of a supported palladium catalyst. Hydrogen is fed to the vessel and the mixture passes through the catalyst bed or beds and exits the hydrogenation zone. The anhydride/acid mixture then may be fractionated by means of a plurality of distillation procedures to separate the anhydride and the acid.

Our novel process and the operation thereof is further illustrated by the following examples. Anhydride/acid mixtures were obtained on two different occasions (separated by about 60 days) from the continuous acetic anhydride manufacturing process described in U.S. Patent 4,374,070. The weight percent ranges of the components of these mixtures were:

| | |
|---|---|
| Acetic anhydride | 50.0-55.0 |
| Acetic acid | 35.0-39.0 |
| Methyl iodide | 0.7-1.0 |
| Methyl acetate | 7.0-9.0 |
| Acetone | 1.0-1.5 |
| Ethylidene diacetate | 0.3-0.5 |
| Acetyl iodide | 120-170 ppm I⁻ |

The mixtures are used in the following procedure without delay.

Each of the mixtures was distilled in an apparatus designed to completely remove all the low boiling constituents. This was accomplished by feeding 1100 mL/hour of the mixture to a laboratory distillation unit which consisted of (1) a heated, 500-mL flask (the base), followed in succession by (2) a 20-tray, 2.54-cm diameter, vacuum-jacketed (silvered) Oldershaw column, (3) a feed section, (4) a 10-tray, 2.54-cm diameter, vacuum-jacketed (silvered) Oldershaw column and, finally, (5) a vapor take-off which was connected to a condensor to prevent vapor losses and to measure the quantity of overhead distillate.

The anhydride/acid mixture was fed at the above-described feed section and the heating of the base flask was adjusted as necessary so that 200 mL of distillate were collected per hour. Aqueous hydrogen peroxide (35%) was fed to the base at the rate of 1.1 mL/hour hydrogen peroxide. The liquid in the base flask was maintained at a level of 250-350 mL by pumping excess liquid to the hydrogen treatment unit.

The hydrogen treatment unit consisted of a 91.44-cm long, 2.54-cm diameter, quartz tube, maintained at approximately 90-125°C, fitted with a 0.635 cm quartz thermal well containing a thermocouple used to monitor temperature and detect any unanticipated exothermic heating or excessive cooling. Optionally, the tube may be insulated or heated to avoid excessive cooling. The tube was filled with 25 g of granulated catalyst which should cover the thermocouple. The top of the tube was fitted with an inlet for hydrogen and the liquid from the above-mentioned base flask. The bottom was connected to a 500 mL, 3-necked flask fitted with a corrosion probe (Petrolite Instruments IN-8000 E/R Portable Electrical Resistance Monitor), a gas outlet and a liquid outlet.

The hydrogen treatment unit was operated by establishing a purge of hydrogen at atmospheric pressure and flowing the liquid over the catalyst bed. The volume of the flask at the bottom of the hydrogen treatment unit was maintained at a level which was sufficient to completely immerse the corrosion probe and the excess liquid was either overflowed or pumped to a subsequent distillation column. Any unconsumed hydrogen was vented via the gas outlet.

The liquid from the hydrogen treatment unit was fed to a distillation column consisting of a vacuum column operated at 100-150 torr and constructed by stacking (1) a heated 500 mL, 3-necked flask (the base) fitted with an outlet port and a thermometer, followed in succession by (2) a 5-tray, 2.54-cm diameter, vacuum jacketed (silvered) Oldershaw column, (3) a feed section, (4) a vacuum-jacketed (silvered), 10-tray, 2.54-cm diameter Oldershaw column, (5) an electrically-timed mechanical liquid product takeoff, (6) a 5-tray, 2.54-cm diameter, vacuum-jacketed (silvered) Oldershaw column, (7) an electrically-timed mechanical liquid product takeoff and, finally, (8) a vented condensor connected to a vacuum regulating device.

Liquid was fed to the distillation column at its feed section after passing the liquid through a steam-jacketed preheater supplied with atmospheric steam using the vacuum to transfer the material and a valve to regulate the flow at 800 mL/hour as determined by the amounts of the takeoffs from the distillation column. The column was operated so that the takeoff from the top of the column was approximately 100 mL/hour and the takeoff from the midpoint of the column was approximately 600 mL/hour. The liquid contents of the base flask were maintained at a level sufficient to cover the thermometer bulb and portions thereof were removed by vacuum. The midpoint takeoff consisting of the desired acetic anhydride/acetic acid mixture was fractionated using a vacuum-jacketed (silvered), 20-tray, 2.54-cm diameter Oldershaw column to obtain the purified acetic anhydride and acetic acid.

Both fractions were analyzed for iodine and inorganic iodide by visible spectrophotometry using a Technicon Autoanalyzer II (continuous flow analysis). The analytical procedure is based on the inverse colorimetric method in which iodine catalyzes the reduction of ceric ions by arsenious acid with the decrease of the yellow ceric ion at 420 nm being proportional to iodide concentration. See E.B. Sanbell and J.M. Kolthoff, **J. Amer. Chem. Soc.,** 1934, **56**, 1426 and V.W. Truesdell and P.J. Smith, **Analyst**, Feb. 1975, pp.111-123. Both fractions also were tested for reducing substances and color according to the procedures described hereinabove.

The results achieved in each of the examples are shown in Table I in which the corrosion rate is given in microns per year, iodine content is given in parts per billion, reducing substances content of acetic anhydride is given in minutes and color of acetic acid is given according to the Platinum-Cobalt Color Standards set forth in ASTM 1209-84. The Control example gives values for a low boiler-depleted anhydride/acid mixture which was not submitted to either a peroxide or hydrogen treatment. The Control mixture was used in Examples 1 and 2 and in Comparative Examples C-1 through C-6. Another anhydride/acid mixture was used in Example 3 and Comparative Example C-6. The acetic anhydride recovered in each of the examples (including the comparative examples) passed the above-described color specification. The acetic acid recovered in each of the examples met the reducing substances specification described hereinabove.

### EXAMPLES 1, 2 and 3

Hydrogen peroxide was added to the low boiler-depleted mixture at 130°C and the peroxide-treated mixture was dripped with a cocurrent of hydrogen through a trickle bed reactor, maintained at approximately 120°C, containing 1.0% palladium on carbon catalyst.

The described peroxide-hydrogen treatment was repeated, using the same anhydride/acid mixture. The results reported in Table I show that the purification process of this invention consistently provides acetic anhydride and acetic acid which meets the above-described specifications set by users of the materials. In Table I, corrosion is given in microns per year (ND = none detected), iodine values are parts per billion, reducing substances values are minutes and color values are determined in accordance with ASTM D 1209-84.

### COMPARATIVE EXAMPLE 1

Example 1 was repeated except that the palladium on carbon catalyst was replaced with quartz chips and argon rather than hydrogen was fed to the modified trickle bed reactor. The modified trickle bed reactor was used to provide a peroxide-anhydride/acid mixture contact time, prior to further refining, essentially the same as the contact time in Example 1 and in the processes described in the prior art.

### COMPARATIVE EXAMPLES 2

Example 1 was repeated except that the palladium on carbon catalyst was replaced with quartz chips. This procedure was performed a second time using an anhydride/acid mixture obtained on a different day from the acetic anhydride manufacturing process referred to above. Comparative Examples 1 and 2 show that a peroxide treatment followed by a hydrogen treatment without a supported palladium catalyst gives improved results but is still inadequate to achieve commercial grade criteria. These examples also demonstrate that processes which do not use a supported palladium catalyst do not give consistently reproducible results.

### COMPARATIVE EXAMPLE 3

Example 1 was repeated except that the peroxide addition step was omitted, i.e., the distilled, low boiler-depleted anhydride/acid mixture was dripped directly through the trickle bed reactor. The results reported in Table I show that the iodine content and the color of the acetic acid refined from the mixture is totally unacceptable.

### COMPARATIVE EXAMPLE 4

Example 1 was repeated except that the palladium on carbon catalyst was replaced with a 1% ruthenium on carbon catalyst. This example shows that a different supported noble metal catalyst gives results substantially inferior to those obtained by the use of the palladium catalyst according to our invention.

### COMPARATIVE EXAMPLE 5

Comparative Example 3 was repeated except that the palladium catalyst was replaced with a 1% ruthenium on carbon catalyst.

**TABLE I**

| Example | Corrosion Rate | Product Analyses | | | |
|---|---|---|---|---|---|
| | | Acetic Anhydride | | Acetic Acid | |
| | | Iodine | Reducing Substances | Iodine | Color |
| Control | 3600 | 1476 | 13 | 937 | 30 |
| 1 | ND | 16.5 | >30 | - | 5 |
| 2 | ND | 12.8 | >30 | 19.9 | 5 |
| 3 | ND | 9.3 | >30 | 9.3 | 5 |
| C-1 | 3700 | 29.0 | 19 | 47.3 | 5 |
| C-2 | ND | 31.0 | 22 | 157 | 5 |
| C-3 | ND | 28.0 | 15 | 404 | 60 |
| C-5 | 2200 | 59.0 | >25 | 6.5 | 5 |
| C-6 | ND | 47.0 | 14 | 16 | 80 |

The reducing substances test used to determine the values reported herein consisted of the following procedure: Just enough 0.1 N aqueous potassium permanganate is added to 100 mL of dilute sulfuric acid solution (10 mL concentrated sulfuric acid per 1 L of solution) to produce a faint pink color. Acetic anhydride (2 mL) is added to and mixed with the dilute sulfuric acid solution followed by the addition of 0.1. mL of 0.1 N potassium permanganate. The time (in minutes up to a maximum of 30 minutes) required for the disappearance of the pink color is the reducing substances test value.

The invention has been described in detail with particular reference to preferred embodiments thereof, but it will be understood that variations and modification can be effected within the spirit and scope of the invention.

## Claims

1. Process comprising contacting a mixture of acetic anhydride and acetic acid, obtained by contacting a mixture comprising methyl iodide and methyl acetate and/or dimethyl ether with carbon monoxide, with (1) a peroxide and (2) hydrogen in the presence of a supported palladium catalyst and subsequently separating the mixture to obtain acetic anhydride and acetic acid, each of which (i) contains 20 parts per billion or less iodine, (ii) has a reducing substances test value of 30 or more minutes and (iii) a color value of 10 or less as determined by ASTM D 1209-84.

2. Process according to Claim 1 wherein the peroxide is hydrogen peroxide.

3. Process according to Claim 1 wherein the mixture of acetic anhydride and acetic acid is contacted with (1) hydrogen peroxide at a temperature of about 50 to 200°C and a pressure of about 0.05 to 10 bar and (2) hydrogen at a temperature of about 25 to 225°C at a pressure of about 0.05 to 50 bar in the presence of a palladium on carbon catalyst.

4. Process according to claim 1 wherein the mixture of acetic anhydride and acetic acid is contacted first with (1) hydrogen peroxide or solutions thereof at a temperature of about 110 to 150°C and a pressure of about 0.5 to 2.5 bar and then with (2) hydrogen at a temperature of about 110 to 150°C at a pressure of about 0.5 to 2.5 bar in the presence of a palladium on carbon catalyst.

## Patentansprüche

1. Verfahren umfassend das Inkontaktbringen einer Mischung aus Essigsäureanhydrid und Essigsäure, erhalten durch Kontaktierung einer Mischung umfassend Methyliodid und Methylacetat und/oder Dimethylether mit Kohlenscoffmonoxid, mit (1) einem Peroxid und (2) Wasserstoff in Gegenwart eines trägergestützten Palladiumkatalysatoren und anschließende Auftrennung der Mischung, um Essigsäureanhydrid und Essigsäure zu erhalten, wobei jedes von beiden (i) 20 ppB oder weniger Iod enthält, (ii) einen Testwert auf reduzierende Substanzen von 30 oder mehr Minuten und (iii) einen Farbwert von 10 oder geringer, bestimmt durch ASTM D 1209-84, hat.

2. Verfahren nach Anspruch 1, wobei das Peroxid Wasserstoffperoxid ist.

3. Verfahren nach Anspruch 1, wobei die Mischung aus Essigsäureanhydrid und Essigsäure mit (1) Wasserstoffperoxid bei einer Temperatur von etwa 50 bis 200°C und einem Druck von etwa 0,05 bis 10 bar und (2) Wasserstoff bei einer Temperatur von etwa 25 bis 225°C bei einem Druck von etwa 0.05 bis 50 bar in Gegenwart eines Palladium-auf-Kohlenstoff-Katalysatoren in Kontakt gebracht wird.

4. Verfahren nach Anspruch 1, wobei die Mischung aus Essigsäureanhydrid und Essigsäure zunächst mit (1) Wasserstoffperoxid oder Lösungen davon bei einer Temperatur von etwa 110 bis 150°C und bei einem Druck von etwa 0,5 bis 2,5 bar in Kontakt gebracht wird und dann mit (2) Wasserstoff bei einer Temperatur von etwa 110 bis 150°C bei einem Druck von etwa 0,5 bis 2,5 bar in Anwesenheit eines Palladium-auf-Kohlenstoff-Katalysatoren.

## Revendications

1. Procédé comprenant la mise en contact d'un mélange d'anhydride acétique et d'acide acétique, obtenu en mettant en contact un mélange comprenant de l'iodure de méthyle et de l'acétate de méthyle et/ou de l'éther diméthylique avec du monoxyde de carbone, avec (1) un peroxyde et (2) de l'hydrogène en présence d'un catalyseur au palladium supporté et en séparant ensuite le mélange pour obtenir de l'anhydride acétique et de l'acide acétique, dont chacun (i) contient 20 parties par milliard ou moins d'iode, (ii) a une valeur de test de substances réductrices de 30 minutes ou plus et (iii) une valeur de couleur de 10 ou moins telle que déterminée par ASTM D 1209-84.

2. Procédé selon la revendication 1 dans lequel le peroxyde est du peroxyde d'hydrogène.

3. Procédé selon la revendication 1 dans lequel le mélange d'anhydride acétique et d'acide acétique est mis en contact avec (1) du peroxyde d'hydrogène à une température d'environ 50 à 200 °C et à une pression d'environ 0,05 à 10 bars et (2) de l'hydrogène à une température d'environ 25 à 225 °C à une pression d'environ 0,05 à 50 bars en présence d'un catalyseur palladium sur carbone.

4. Procédé selon la revendication 1 dans lequel le mélange d'anhydride acétique et d'acide acétique est mis en contact d'abord avec (1) du peroxyde d'hydrogène ou des solutions de celui-ci à une température d'environ 110 à 150 °C et à une pression d'environ 0,5 à 2,5 bars et ensuite avec (2) de l'hydrogène à une température d'environ 110 à 150 °C à une pression d'environ 0,5 à 2,5 bars en présence d'un catalyseur palladium sur carbone.
